# EUROPEAN PATENT APPLICATION

(11) **EP 1 749 478 A1**
(43) Date of publication of application: **07.02.2007**
(21) Application number: 05737152.8
(22) Date of filing: 02.05.2005
(51) Int. Cl.: A61B 8/14

(54) **ULTRASONIC DIAGNOSIS APPARATUS**

(30) Priority: 25.05.2004 JP 2004155078
(71) Applicant: MATSUSHITA ELECTRIC INDUSTRIAL CO., LTD., Kadoma-shi, Osaka 571-8501 (JP)
(72) Inventor:
(74) Representative: Schorr, Frank Jürgen
(86) International application number: PCT/JP2005/008278
(87) International publication number: WO 2005/115250

(57) **Abstract**

An ultrasonic diagnostic apparatus includes a transducer array 1 in which a plurality of transducers for transmitting an ultrasonic wave to a subject and receiving a reflected wave therefrom are arrayed, and delay addition units 4 to 9 for performing parallel reception by adding a delay time to signals from the transducer array. The apparatus further includes a deflection angle control unit 14 that performs control so that an angle formed between a plurality of directions of reception directivities in the parallel reception decreases as a deflection angle of a transmission beam transmitted from the transducer array increases. Thus, the ultrasonic diagnostic apparatus that keeps relative sensitivities in the parallel reception uniform irrespective of the deflection angle of the ultrasonic transmission beam is provided.

## Description

### Technical Field

The present invention relates to an ultrasonic diagnostic apparatus that includes a transducer array for scanning a subject.

### Background Art

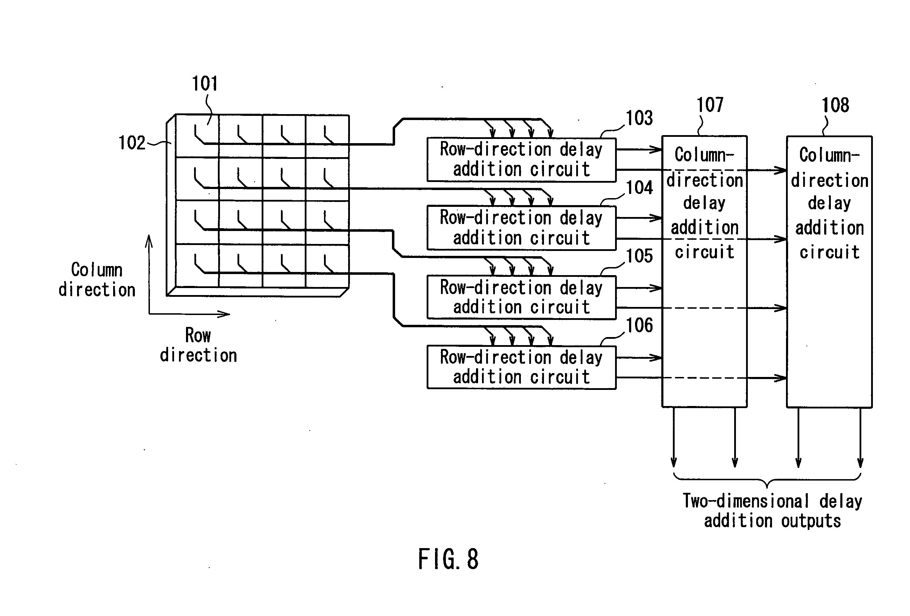

A conventional ultrasonic diagnostic apparatus includes, as shown in FIG. 8, a two-dimensional array 102 in which transducers 101 are arrayed, row-direction delay addition circuits 103 to 106, and column-direction delay addition circuits 107 and 108. The row-direction delay addition circuits 103 to 106 delay and add signals detected by the transducers 101 arrayed in the row direction in the two-dimensional array 102. The column-direction delay addition circuits 107 and 108 delay and add signal groups that have been obtained by a delaying and adding operation conducted by the row-direction delay addition circuits 103 to 106. By so doing, the parallel reception in the row and column directions is realized with a small scale circuit (see patent document 1).
Patent document 1: JP 2000-254120 A

### Disclosure of Invention

### Problem to be Solved by the Invention

The conventional ultrasonic diagnostic apparatus has a problem in that relative sensitivities in the parallel reception are made non-uniform due to a deflection angle of a transmission beam.

To solve the foregoing problem of the prior art, it is an object of the present invention to provide an ultrasonic diagnostic apparatus that is configured so that the relative sensitivities in the parallel reception can be kept uniform irrespective of the deflection angle of the ultrasonic transmission beam.

### Means for Solving Problem

An ultrasonic diagnostic apparatus of the present invention includes: a transducer array in which a plurality of transducers for transmitting an ultrasonic wave to a subject and receiving a reflected wave therefrom are arrayed; a delay addition unit for performing parallel reception by carrying out a delay addition operation with respect to reception signals obtained by the transducer array; and a deflection angle control unit for controlling a deflection angle for reception according to a setting for the delay addition operation carried out by the delay addition unit. The ultrasonic diagnostic apparatus is characterized in that the deflection angle control unit narrows an angle formed between a plurality of directions of directivities of reception in the parallel reception as a deflection angle of a transmission beam transmitted from the transducer array increases.

With this configuration, the non-uniformity of relative sensitivities in the parallel reception due to the deflection angle of the transmission beam can be reduced.

The foregoing apparatus may be configured further so as to include a correction unit for performing control for changing sensitivity correction amounts for a plurality of reception signals in the parallel reception in a manner such that a decrease in a relative sensitivity in transmission-reception due to an increase in the deflection angle of the transmission beam is compensated.

The foregoing apparatus may be configured further so that the correction unit performs correction such that any of the plurality of reception signals received in a state such that angles between respective directions of reception directivities in the parallel reception and a direction of a directivity of the transmission beam (hereinafter referred to as transmission directivity in some cases) are equal to one another have relative sensitivities equal to one another.

With this configuration, the sensitivity correction can be carried out easily.

The foregoing deflection angle control unit may be configured further so as to perform control such that a difference between a deflection angle determining a direction of the transmission beam and a deflection angle determining a next direction of the transmission beam decreases as the deflection angle of the transmission beam increases.

The foregoing apparatus may be configured further so that the plurality of transducers are arrayed at least two-dimensionally, and a plurality of points at which the transmission beam crosses a projection face form lattice points that are arrayed two-dimensionally at uniform intervals.

With such configurations, in the case where a plurality of transducers arrayed two-dimensionally are used, differences between the deflection angles of neighboring directions of the transmission beam are narrowed as the deflection angle of the transmission beam increases. Consequently, the relative sensitivities in the parallel reception can be kept uniform irrespective of the deflection angle.

### Effects of the Invention

According to the present invention, it is possible to provide an ultrasonic diagnostic apparatus that is configured so that the relative sensitivities in the parallel reception can be kept uniform irrespective of the deflection angle of the ultrasonic transmission beam.

### Brief Description of Drawings

[FIG. 1] FIG. 1 is a block diagram showing a configuration of a reception front end of an ultrasonic diagnostic apparatus according to Embodiment 1.
[FIG. 2] FIG. 2 is a diagram showing a configuration of a sub-array according to Embodiment 1.
[FIG. 3] FIG. 3 is a graph showing azimuth angle dependence of a relative sensitivity in the parallel reception in Embodiment 1.
[FIG. 4A] FIG. 4A is a graph showing a relative sensitivity of reception in the case where the deflection angle of the transmission beam is 0° in Embodiment 1.
[FIG. 4B] FIG. 4B is a graph showing a relative sensitivity of reception in the case where the deflection angle of the transmission beam is 30° in Embodiment 1.
[FIG. 5A] FIG. 5A is a diagram showing angles formed between reception directivities in the parallel reception in Embodiment 1. '
[FIG. 5B] FIG. 5B is a diagram showing differences between deflection angles of the transmission beam in Embodiment 1.
[FIG. 6A] FIG. 6A is a side view showing an ultrasonic beam transmitted from the transducer array according to Embodiment 2.
[FIG. 6B] FIG. 6B is a top view showing the ultrasonic beam according to Embodiment 2.
[FIG. 7] FIG. 7 is a diagram showing the centers of a transmission beam and reception sensitivities in the parallel reception of an ultrasonic diagnostic apparatus according to Embodiment 3.
[FIG. 8] FIG. 8 is a block diagram showing a configuration of a reception front end of a conventional ultrasonic diagnostic apparatus.

### Description of Reference Numerals

- 1: transducer array
- 2: sub-array (SA)
- 3: intra-group processor (IP)
- 4 to 7: first-direction delay addition circuit
- 8 to 9: second-direction delay addition circuit
- 10 to 13: correction circuit
- 14: deflection angle control circuit
- 101: transducer
- 102: two-dimensional array
- 103 to 106: row-direction delay addition circuit
- 107,108: column-direction delay addition circuit

### Description of the Invention

The following describes ultrasonic diagnostic apparatuses according to embodiments of the present invention, while referring to the drawings.

### Embodiment 1

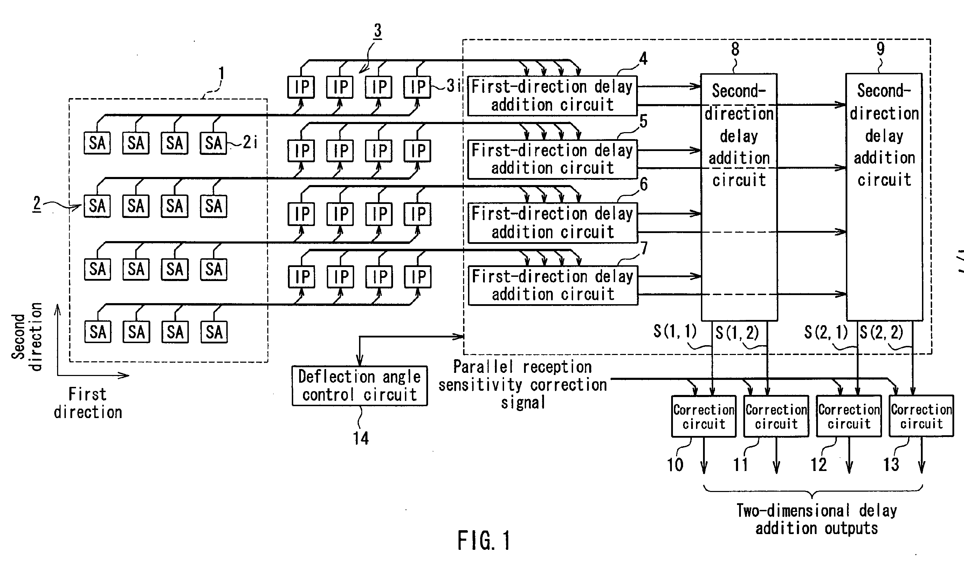

FIG. 1 is a block diagram showing principal members of a reception front end of an ultrasonic diagnostic apparatus according to Embodiment 1. A transducer array 1 is formed by arraying a plurality of sub-arrays (SA) 2 in a first direction and a second direction. In FIG. 1, the sub-arrays (SA) 2 are connected respectively with elements of intra-group processors (IP) 3, which will be described later. An output of the i-th sub-array (SA) 2i is fed to the intra-group processor (IP) 3i.

Outputs of the intra-group processors (IP) 3 are fed to first-direction delay addition circuits 4 to 7 (first-direction delay addition units). A plurality of parallel reception outputs of the first-direction delay addition circuit 4 are fed to second-direction delay addition circuits 8 and 9 (second-direction delay addition units). Likewise, a plurality of outputs of first-direction delay addition circuits 5 to 7 are fed to the second-direction delay addition circuits 8 and 9. A deflection angle control circuit 14 (deflection angle control unit) incorporates software for correcting a deflection angle of parallel reception, so as to determine delay addition values that are to be used in delaying and adding operations performed by the first-direction delay addition circuits 4 to 7 and the second-direction delay addition circuits 8 and 9.

A plurality of parallel reception outputs S(1,1) and S(1,2) of the second-direction delay addition circuit 8 are fed to correction circuits 10 and 11, respectively. A plurality of parallel reception outputs S(2,1) and S(2,2) of the second-direction delay addition circuit 9 are fed to correction circuits 12 and 13, respectively. The correction circuits 10 to 13 (correction units) subject the parallel reception outputs to sensitivity correction, according to a parallel reception sensitivity correction signal fed thereto. Outputs from the correction circuits 10 to 13 are two-dimensional delay addition outputs.

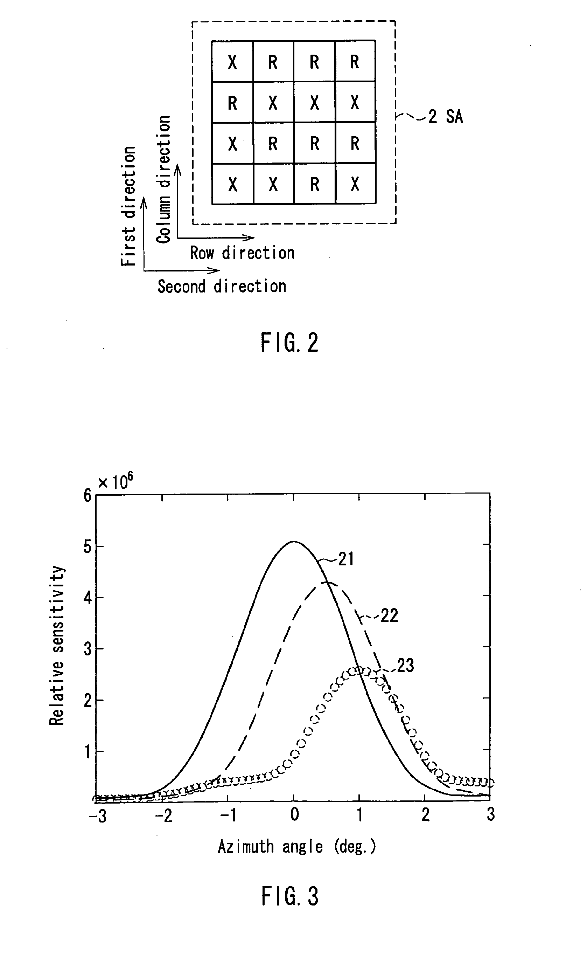

FIG. 2 is a diagram showing the configuration of the sub-array (SA) 2. The sub-array (SA) 2 is composed of transducers (X) for transmission and transducers (R) for reception, and these transducers are arrayed in column and row directions. The column direction coincides with the first direction, while the row direction coincides with the second direction.

FIG. 3 is a graph showing an example of azimuth angle dependence of the relative sensitivity in the parallel reception. Curves 21, 22, and 23 in FIG. 3 indicate relative sensitivities in transmission-reception with respect to different directions of the reception directivities with respect to a transmission beam, respectively. The curve 21 indicates the relative sensitivity in transmission-reception in the case where the direction of the transmission directivity and the direction of the reception directivity coincide with each other. The curve 22 indicates the relative sensitivity in transmission-reception in the case where the direction of the reception directivity is deviated by 1° from the direction of the transmission directivity due to the parallel reception, which causes the direction of the transmission-reception directivity to deviate by 0.5°. The curve 23 indicates the relative sensitivity in the case where the direction of the reception directivity is deviated by 2° from the direction of the transmission directivity due to the parallel reception, which causes the direction of the transmission-reception directivity to deviate by 1°. In other words, the curves 22 and 23 indicate the sensitivities in the case where the direction of the transmission directivity and the direction of the reception directivity do not coincide with each other. Besides, it shows that as the deviation of the direction of the reception directivity from the direction of the transmission directivity increases, the relative sensitivity decreases.

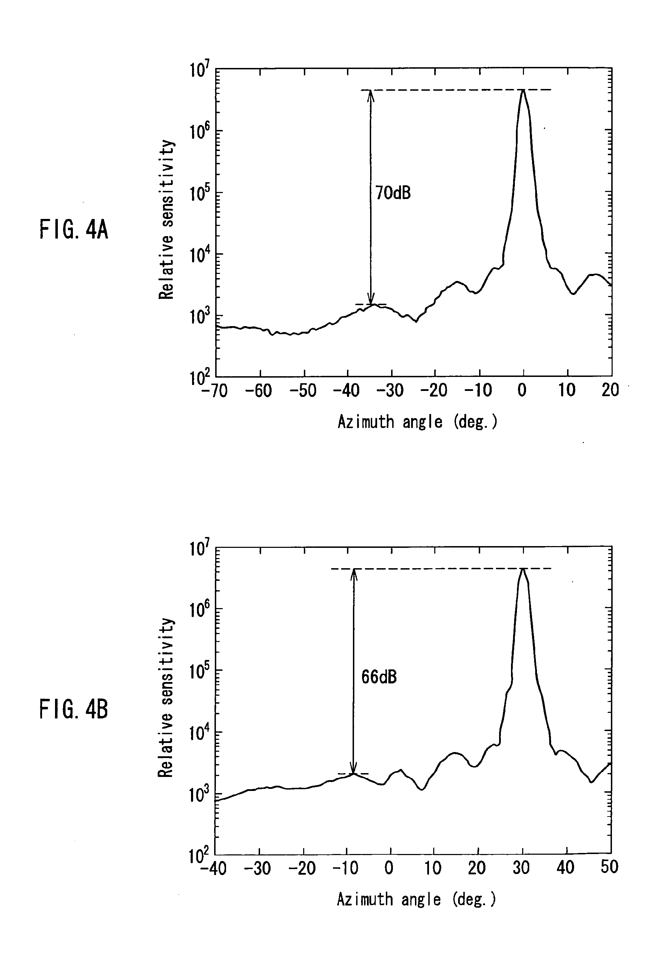

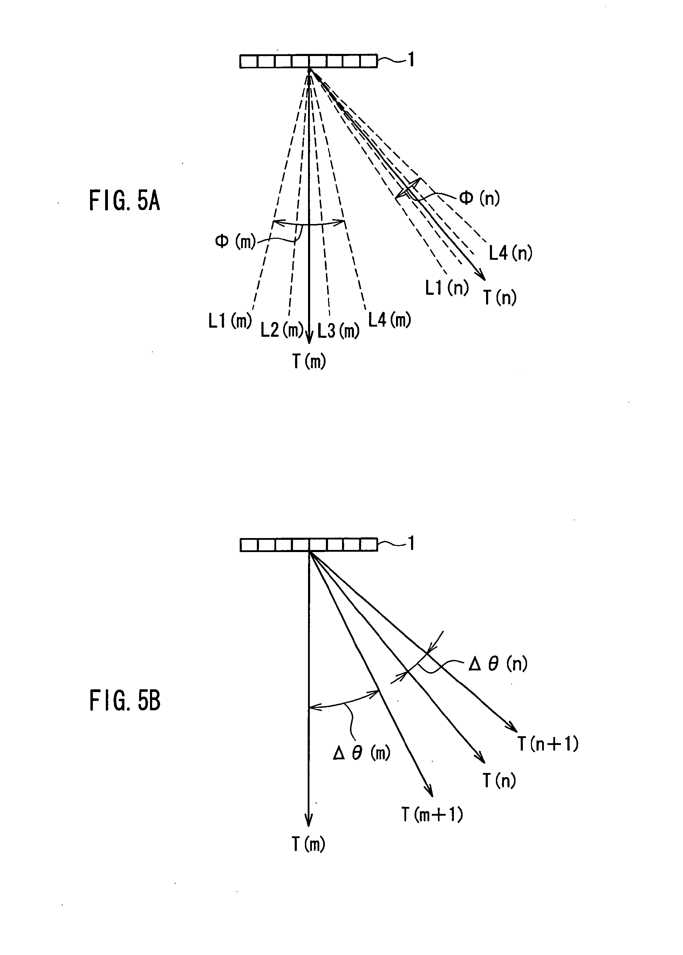

FIG. 4A is a graph showing an example of the relative sensitivity in the case where the deflection angle of the transmission beam is 0°, and FIG. 4B is a graph showing an example of the relative sensitivity in the case where a deflection angle of the transmission beam is 30°. FIG. 5A is a view showing angles formed between directions of directivities in the parallel reception. FIG. 5B is a view showing differences between deflection angles of transmission beams. T(m) indicates a direction of a directivity of a transmission beam that is not deflected, while L1(m) to L4(m) indicate directions of directivities in the parallel reception corresponding to the direction T(m) of the directivity of the transmission beam. T(n) indicates a direction of a directivity of a transmission beam that is deflected, while L1(n) to L4(n) indicate directions of directivities in the parallel reception corresponding to the direction T(n).

Operations of a reception front end of the ultrasonic diagnostic apparatus configured as described above are described below, with reference to FIGS. 1 to 5B.

First, a transmission ultrasonic pulse is transmitted from the transducers (X) for transmission in the sub-arrays (SA) 2 to a region of interest. Reception signals from the transducers (R) for reception in the sub-arrays (SA) 2 are phased with one another by the intra-group processors (IP) 3. Outputs of the intra-group processors (IP) 3 corresponding to the sub-arrays (SA) 2 aligned in the first direction are fed in a batch to a corresponding one of the first-direction delay addition circuits 4 to 7.

The first-direction delay addition circuits 4 to 7 output parallel reception signals having directivities toward a region of interest, which are directed in a plurality of directions deviated with respect to the first direction. The second-direction delay addition circuits 8 and 9 generate reception delay times so that the directions of directivities are deviated through infinitesimal angles, respectively, with respect to the second direction so as to correct delay times for the parallel reception signals outputted by the first-direction delay addition circuits 4 to 7, and output parallel reception signals. The parallel reception signals outputted from the second-direction delay addition circuits 8 and 9 are subjected to correction of their signal intensities by the correction circuits 10 to 13. As shown in FIG. 3, in the parallel reception, a relative sensitivity in the transmission-reception varies significantly according to a difference between the direction of the directivity of the transmission beam and the direction of the reception directivity. Therefore, in the case where the difference between the direction of the transmission directivity and the direction of the reception directivity is varied according to the deflection angle of the transmission, it is necessary to correct a change in the relative sensitivity with use of the correction circuits 10 to 13.

As shown in FIG. 4A, in the case where the deflection angle of the direction of the transmission directivity is 0°, a difference between a peak and a side lobe of the relative sensitivity, that is, a dynamic range, is approximately 70 dB. On the other hand, as shown in FIG. 4B, in the case where the deflection angle of the direction of the transmission directivity is 30°, the relative sensitivity has a dynamic range of approximately 66 dB.

Therefore, the difference between the directions of the transmission directivity and the reception directivity in the parallel reception when the deflection angle is 30° is set smaller than the difference when the deflection angle is 0°, whereby the relative sensitivity in the transmission-reception is enhanced as shown in FIG. 3. By so doing, a decrease in the relative sensitivity in the parallel reception, which is indicated by the main lobe, is reduced, which reduces deterioration of the dynamic range. Accordingly, a difference between the dynamic range of the relative sensitivity when the deflection angle of the transmission beam is 0° and the dynamic range of the relative sensitivity when the deflection angle of the transmission beam is 30° can be reduced.

In FIG. 5A, an angle φ represents an angle formed between directions L1 and L4 of directivities in the parallel reception corresponding to a direction T of a directivity of a transmission beam. An angle φ(m) corresponding to a direction T(m) of the directivity of the transmission beam when the deflection angle is 0° is larger than an angle φ(n) corresponding to a direction T(n) of the directivity of the transmission beam when the beam is deflected. Therefore, as shown in FIG. 5B, an angle Δθ(m) formed between the directions T(m) and T(m+1) of the directivities of the transmission beam with a small deflection angle of transmission is set to be larger than an angle Δθ(n) formed between the directions T(n) and T(n+1) of the directivities of the transmission with a large deflection angle of transmission.

In such a reception front end of the ultrasonic diagnostic apparatus in the present embodiment, the deflection angle control circuit 14 controls the angle formed between a plurality of directions of the reception directivities in the parallel reception to become narrower as the deflection angle for the transmission increases. Then, differences of the relative sensitivities in the transmission-reception due to the variety of the angles formed between the directions of the reception directivities in the parallel reception are corrected by the correction circuits 10 to 13, whereby an image with uniform relative sensitivities can be obtained.

Further, a problem of an increase in a difference between the directions of the reception directivities corresponding to neighboring directions of the transmission directivities also can be solved by narrowing the angles formed between the plurality of directions of the reception directivities in the parallel reception as the deflection angle in the transmission increases.

It should be noted that examples of the method for correcting the deflection angle in the parallel reception include (1) a method in which the correction value is determined by computation, (2) a method in which correction values are stored in a data table for correction and a suitable correction value is selected therefrom, (3) a method in which the methods (1) and (2) are used in combination, and the like. Apart from the case where the deflection angle control circuit 14 is configured to use such a method, each of the first-direction delay addition circuits 4 to 7 and the second-direction delay addition circuits 8 and 9 may be configured to use such a method.

### Embodiment 2

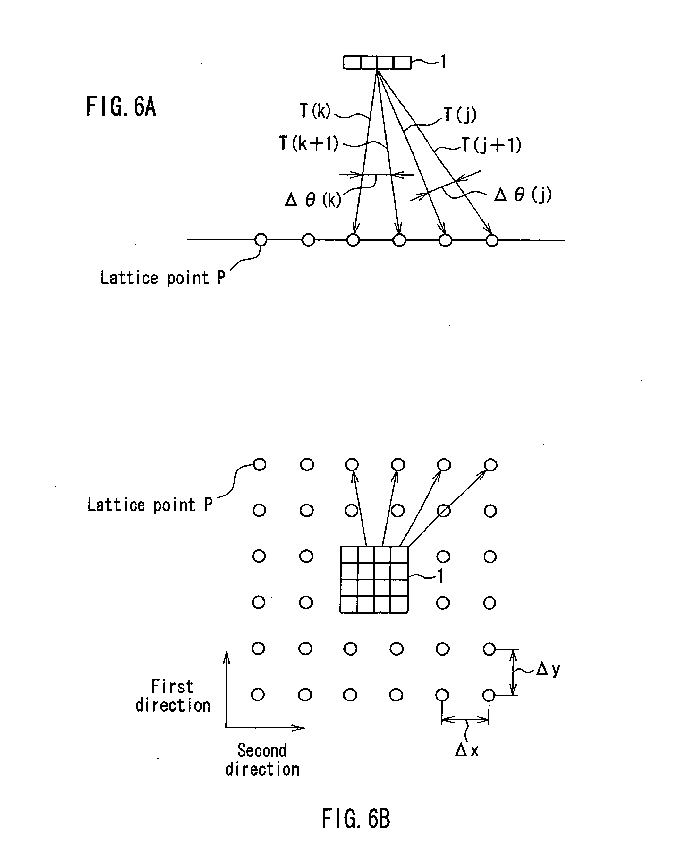

Intervals of directions of a transmission beam of an ultrasonic diagnostic apparatus in Embodiment 2 are shown in FIGS. 6A and 6B. It should be noted that members having the same configurations and functions as those shown in FIG. 5B, which is referred to regarding Embodiment 1, are designated by the same reference signs and numerals, and descriptions of the same are omitted. Further, the other constituent members not shown in FIG. 6 are identical to those shown in FIG. 1.

FIG. 6A is a side view of the transducer array 1, in which a projection face is disposed substantially in parallel with the transducer array 1 and a transmission beam crosses the projection face at lattice points p indicated with circles. FIG. 6B is a top view of the configuration shown in FIG. 6A.
It should be noted that the projection face may be a planar face perpendicular to a beam at the center of scanning.

Regarding the intervals of the transmission beam in the ultrasonic diagnostic apparatus thus configured, an operation thereof is described below with reference to FIG. 6.

First, in FIG. 6B, the lattice points p are arranged two-dimensionally at intervals Δx in the first direction and intervals Δy in the second direction. In FIG. 6A, an angle Δθ(k) formed between a direction T(k) of a directivity of a transmission beam and a direction T(k+1) of the same at a small deflection angle of transmission is set to be larger than an angle Δθ(j) formed between a direction T(j) of a directivity of the transmission beam and a direction T(j+1) of the same at a large deflection angle of the transmission beam. Further, an angle formed between directions of reception directivities in the parallel reception that correspond to the direction T(k) is set to be larger than an angle formed between directions of reception directivities in the parallel reception that correspond to the direction T(j).

As described above, in the ultrasonic diagnostic apparatus according to Embodiment 2, the lattice points p at which the transmission beam crosses the projection face are arrayed two-dimensionally at uniform intervals, i.e., at intervals Δx in the first direction and intervals Δy in the second direction. Therefore, as the deflection angle of the transmission beam increases, the angles formed between directions of the reception directivity in the parallel reception decrease, whereby an excellent image with uniform relative sensitivities can be obtained.

### Embodiment 3

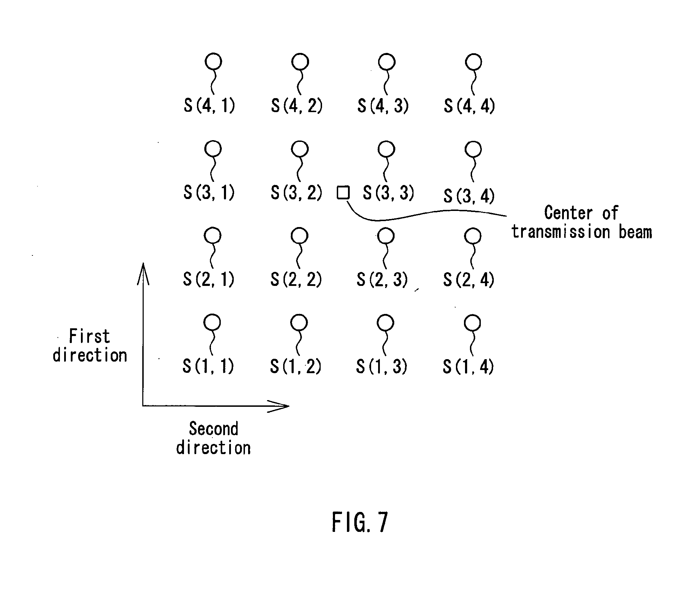

The center of a transmission beam and the centers of reception sensitivities in the parallel reception of an ultrasonic diagnostic apparatus according to Embodiment 3 are shown in FIG. 7. It should be noted that in FIG. 7, members having the same configurations and functions as those shown in FIG. 1, which is referred to regarding Embodiment 1, are designated by the same reference signs and numerals, and descriptions of the same are omitted. Further, the other constituent members not shown in FIG. 7 are identical to those shown in FIG. 1.

In FIG. 7, a mark □ indicates the center of the transmission beam, and marks o indicate the centers of reception sensitivities in the parallel reception. The centers of the reception sensitivities in the parallel reception are designated with codes S(x,y) (1≤x≤4, 1≤y≤4) of parallel reception signals corresponding thereto, respectively.

The operations of the transmission beam and the parallel reception beam in the ultrasonic diagnostic apparatus thus configured are described below, with reference to FIG. 7.

The parallel reception signals are classified into three groups. In FIG. 7, the first group is composed of S(1,1), S(1,4), S(4,1), and S(4,4), and respective signals received therefrom are reception signals received in a state such that distances from the center of the transmission beam to the respective positions are equal to one another and angles formed between respective directions of the reception directivities in the parallel reception and the direction of the directivity of the transmission beam are equal to one another. Therefore, these signals are corrected by the correction circuits 10 to 13 by using one and the same parallel reception sensitivity correction signal.

Likewise, the second group is composed of S(2,2), S(2,3), S(3,2), and S(3,3), and respective signals received therefrom are reception signals received in a state such that distances from the center of the transmission beam to the respective positions are equal to one another and angles formed between respective directions of the reception directivities in the parallel reception and the direction of the directivity of the transmission beam are equal to one another. Therefore, these signals are corrected by the correction circuits 10 to 13 by using one and the same parallel reception sensitivity correction signal. Further, the third group is composed of S(1,2), S(1,3), S(2,1), S(2,4), S(3,1), S(3,4), S(4,2) and S(4,3), and respective signals received therefrom are reception signals received in a state such that distances from the center of the transmission beam to the respective positions are equal to one another and angles formed between respective directions of the reception directivities in the parallel reception and the direction of the directivity of the transmission beam are equal to one another. Therefore, these signals are corrected by the correction circuits 10 to 13 by using one and the same parallel reception sensitivity correction signal.

As described above, in the configuration with the transmission beam and the parallel reception beam of the ultrasonic diagnostic apparatus according to Embodiment 3, the sixteen parallel reception signals S(x,y) are divided into three groups. By so doing, the sensitivity correction can be carried out with use of three types of parallel reception sensitivity correction signals, whereby the control is made easier.

### Industrial Applicability

The ultrasonic diagnostic apparatus of the present invention achieves an effect that an image with uniform relative sensitivities can be obtained, and therefore, the foregoing apparatus is advantageous as an ultrasonic diagnostic apparatus including a transducer array for scanning a subject.

## Claims

1. An ultrasonic diagnostic apparatus comprising:
a transducer array in which a plurality of transducers for transmitting an ultrasonic wave to a subject and receiving a reflected wave therefrom are arrayed;
a delay addition unit for performing parallel reception by carrying out a delay addition operation with respect to reception signals obtained by the transducer array; and
a deflection angle control unit for controlling a deflection angle for reception according to a setting for the delay addition operation carried out by the delay addition unit,
wherein the deflection angle control unit narrows an angle formed between a plurality of directions of reception directivities in the parallel reception as a deflection angle of a transmission beam transmitted from the transducer array increases.

2. The ultrasonic diagnostic apparatus according to claim 1, further comprising a correction unit for performing control for changing sensitivity correction amounts for a plurality of reception signals in the parallel reception in a manner such that a decrease in a relative sensitivity in transmission-reception due to an increase in the deflection angle of the transmission beam is compensated.

3. The ultrasonic diagnostic apparatus according to claim 2, wherein the correction unit performs correction such that any of the plurality of reception signals received in a state such that angles between respective directions of reception directivities in the parallel reception and a direction of a directivity of the transmission beam are equal to one another have relative sensitivities equal to one another.

4. The ultrasonic diagnostic apparatus according to any one of claims 1 to 3, wherein the deflection angle control unit performs control such that a difference between a deflection angle determining a direction of the transmission beam and a deflection angle determining a next direction of the transmission beam decreases as the deflection angle of the transmission beam increases.

5. The ultrasonic diagnostic apparatus according to claim 4, wherein
the plurality of transducers are arrayed at least two-dimensionally, and
a plurality of points at which the transmission beam crosses a projection face form lattice points that are arrayed two-dimensionally at uniform intervals.
